(19) Europäisches Patentamt
European Patent Office
Office européen des brevets

(11) **EP 4 522 105 B1**

(12) **EUROPÄISCHE PATENTSCHRIFT**

(45) Veröffentlichungstag und Bekanntmachung des Hinweises auf die Patenterteilung:
**10.06.2026   Patentblatt 2026/24**

(21) Anmeldenummer: **23717960.1**

(22) Anmeldetag: **12.04.2023**

(51) Internationale Patentklassifikation (IPC):
*A61K 8/02* (2006.01)      *A61K 8/34* (2006.01)
*A61K 8/60* (2006.01)      *A61K 8/92* (2006.01)
*A61Q 19/10* (2006.01)      *A61K 8/73* (2006.01)

(52) Gemeinsame Patentklassifikation (CPC):
**A61K 8/60; A61K 8/0295; A61K 8/342; A61K 8/604; A61K 8/73; A61K 8/92; A61Q 19/10**

(86) Internationale Anmeldenummer:
**PCT/EP2023/059487**

(87) Internationale Veröffentlichungsnummer:
**WO 2023/217480 (16.11.2023 Gazette 2023/46)**

(54) **EMULSION MIT FLÜSSIGKRISTALLINEN STRUKTUREN**

EMULSION WITH LIQUID CRYSTALLINE STRUCTURES

ÉMULSION À STRUCTURES CRISTALLINES LIQUIDES

(84) Benannte Vertragsstaaten:
**AL AT BE BG CH CY CZ DE DK EE ES FI FR GB GR HR HU IE IS IT LI LT LU LV MC ME MK MT NL NO PL PT RO RS SE SI SK SM TR**

(30) Priorität:  **12.05.2022   DE 102022204661**

(43) Veröffentlichungstag der Anmeldung:
**19.03.2025   Patentblatt 2025/12**

(73) Patentinhaber: **Beiersdorf AG**
**22529 Hamburg (DE)**

(72) Erfinder:
• **TANG, Wei**
**22529 Hamburg (DE)**
• **FRANCK, Kerstin**
**25451 Quickborn (DE)**
• **XU, Yang**
**20249 Hamburg (DE)**

(74) Vertreter: **Beiersdorf AG**
**Patentabteilung**
**Beiersdorfstraße 1 - 9**
**22529 Hamburg (DE)**

(56) Entgegenhaltungen:
CN-A- 109 528 503

• **DATABASE GNPD [online] MINTEL; 27 October 2011 (2011-10-27), ANONYMOUS: "Facial Mask", XP093064868, retrieved from https://www.gnpd.com/sinatra/recordpage/1660731/ Database accession no. 1660731**
• **ANONYMOUS: "Pigmenthaltige sonnenschutzemulsionen vom typ O/W mit verbesserten eigenschaften", RESEARCH DISCLOSURE, KENNETH MASON PUBLICATIONS, HAMPSHIRE, UK, GB, vol. 435, no. 7, 1 July 2000 (2000-07-01), XP007126375, ISSN: 0374-4353**
• **ANONYMOUS: "Myristyl Alcohol |", 30 June 2021 (2021-06-30), XP093066651, Retrieved from the Internet <URL:https%3A%2F%2Fatamankimya.com%2Fsayfalar.asp%3FLanguageID%3D2%26cid%3D3%26id%3D11%26id2%3D10626> [retrieved on 20230724]**

**Beschreibung**

Kosmetische Produkte dienen im Allgemeinen nicht nur dazu schön und attraktiv auszusehen, sondern sie tragen mit ihrer Wirkung entscheidend zu einem gesteigerten Selbstwertgefühl und zum Wohlbefinden der Menschen bei. Dementsprechend werden die verschiedensten kosmetischen Produkte zur täglichen Reinigung und Pflege der menschlichen Haut eingesetzt.

Hautpflegeprodukte bestehen in der Regel aus Emulsionen. Unter Emulsionen versteht man im Allgemeinen heterogene Systeme, die aus zwei nicht oder nur begrenzt miteinander mischbaren Flüssigkeiten bestehen, die üblicherweise als Phasen bezeichnet werden und bei denen eine der beiden Flüssigkeiten in Form feinster Tröpfchen in der anderen Flüssigkeit dispergiert ist. Äußerlich und mit bloßem Auge betrachtet erscheinen Emulsionen homogen.

Sind die beiden Flüssigkeiten Wasser und Öl und liegen Öltröpfchen fein verteilt in Wasser vor, so handelt es sich um eine Öl-in-Wasser-Emulsion (O/W-Emulsion, z. B. Milch). Der Grundcharakter einer O/W-Emulsion ist durch das Wasser geprägt. Bei einer Wasser-in-Öl-Emulsion (W/O-Emulsion, z. B. Butter) handelt es sich um das umgekehrte Prinzip, wobei der Grundcharakter hier durch das Öl bestimmt wird.

Zur Stabilisierung und zur Verdickung von O/W-Emulsionen werden zumeist Acrylat-basiete Polymere in diese Formulierungen eingearbeitet. Acrylat-basierte Polymere sind Polymere, welche aus Homo- oder Copolymerisation mit Acryl- und/oder Methacrylsäure erhalten werden. Beispiele hierfür sind u.a. Carbomer oder Acrylate Copolymer.

Emulsionen mit Acrylaten sind u.a. aus DE 10148825 A1, DE 19938756 A1 und DE 29924371 U1 bekannt.

Acrylat-basierte Polymere werden insbesondere in tiegelfähigen Emulsionen in Kombination mit Wachskomponenten eingesetzt. Entscheidend bei diesen Formulierungen sind deren rheologische Eigenschaften. So sollen die Formulierungen so viskos sein, dass diese nicht unter Normalbedingungen aus einem geöffneten Tiegel tropfen können. Folglich sind diese Formulierungen nur bedingt fließfähig unter Normalbedingungen. Besonders beliebt sind Tiegelfähige Produkte, die so formuliert sind, dass die Formeln nach Auftragung auf die Haut auf dieser schmelzen und so dem Verbraucher ein besonderes Hautgefühl vermitteln.

Jedoch ist der Einsatz dieser Acrylat-basierten Polymere zunehmend in der Kritik, da deren biologische Abbaubarkeit nicht vollständig geklärt ist.

Problematisch ist der Umstand des der Austausch von Carbomeren dazu führt, dass die Tiegel gerade bei höheren Temperaturen besonders leicht auslaufen. Dieses ist nur dadurch zu kompensieren, dass höher schmelzende Wachskomponenten ein formuliert werden. Das hat jedoch den Nachteil, dass der Hautschmelzeffekt aufgehoben wird. Folglich geht der Produktvorteil verloren.

Aufgabe der vorliegenden Erfindung war es, eine Emulsion bereitzustellen, die die Nachteile des Standes der Technik nicht aufweist und die es erlaubt den Hautschmelzeffekt unter Verhinderung des Auslaufens des Produkts unter erhöhten Temperaturen zu ermöglichen.

Unter erhöhten Temperaturen werden Temperaturen von 45°C verstanden, da diese Temperaturen durch Sonneneinstrahlung bei Lagerung der Produkte erreicht werden können.

Weiterhin beschreibt die DE 2909087 C2 beschreibt ein Verfahren zur Bestimmung der Konsistenz von pastösen Zubereitungen, wie Cremes. Dabei wird der spürbare Druck ermittelt, welcher entsteht, wenn ein Messkörper in einem definierten Verfahren durch die Creme geführt wird. Die damit erhaltene Konsistenz gibt an, welche Kraft aufgewendet werden muss, um den Messkörper durch die Creme zu führen. Dieses ist entscheidend für Produkte, wie Cremes, die in Tiegeln gelagert werden und die Aufnahme der Creme mittels eines Fingers erfolgt. Es ist wünschenswert, dass Formulierungen eine ausreichende Konsistenz aufweisen. So kann die Aufnahme mit dem Finger leicht erfolgen. Liegt eine zu geringe Konsistenz vor, so verschiebt man mit dem Finger nur die Creme und es ist keine leichte Aufnahme möglich.

Ein weiterer Nachteil im Stand der Technik ist, dass die Konsistenz von Cremen und pastösen Emulsionen oft nur mittels Acrylat-basierten Polymere auf einem ansprechenden Niveau erhalten werden kann. Insbesondere bei höheren Temperaturen, wie 45°C, weisen Emulsionen acrylatfreie Emulsionen oftmals eine unzureichende Konsistenz auf.

Der Stand der Technik kennt weiterhin das Dokument CN 109528503 A, welches kosmetische Zubereitungen mit flüssigkristallinen Netzwerken beschreibt. Weiterhin beschreibt das Dokument "Pigmenthaltige sonnenschutzemulsionen vom typ O/W mit verbesserten eigenschaften", RESEARCH DISCLOSURE, KENNETH MASON PUBLICATIONS, HAMPSHIRE, UK, GB, (20000701), vol. 435, no. 7, ISSN 0374-4353 kosmetische Zubereitungne mit Stärken. Jedoch konnten diese Dokumente keinen Hinweis auf die Erfindung geben.

Überraschend konnte die vorliegende Erdfindung die Probleme des Standes der Technik adressieren.

Gegenstand der Erfindung ist eine acrylatfreie kosmetische Emulsion aufweisend

a) Ein Emulgatorsystem, welches in der Emulsion ein flüssigkristallines Netzwerk ausbildet, wobei das Emulgatorsystem

    a. Mindestens ein Fettalkoholglucosid, und

b. Myristylalkohol

umfasst,
b) Mindestens ein Polysaccharid, und
c) Weiterhin mindestens eine Wachskomponente mit einem Schmelzpunkt von weniger als 43°C

dadurch gekennzeichnet, dass

- die Polysaccharide aus den Gruppen der Stärken und/oder Cellulosen gewählt werden und
- Fettalkohole und Fettsäuren aufweisend Alkylketten mit 10 bis 20 Kohlenstoffatomen definitionsgemäß nicht zu den Wachskomponenten zählen.

Acrylatfrei bedeutet definitionsgemäß, dass keine Polymere enthalten sind, welche aus Homo- oder Copolymerisation mit Acryl- und/oder Methacrylsäure erhalten werden. Definitionsgemäß weisen Fettalkohole eine C-10 bis C-20 Kohlenstoffkette auf.

Auch Gegenstand der Erfindung ist die Verwendung der Emulsion der Erfindung zur Bereitstellung eines kosmetischen Produkts.

Vorteilhaft handelt es sich um eine Öl-in-Wasser (O/W) Emulsion.

Sollten nachfolgend Gewichtsprozentangaben (Gew.-%) ohne Bezugnahme auf eine bestimmte Zusammensetzung oder spezifische Mischung angegeben werden, so beziehen sich diese Angaben immer auf das Gesamtgewicht der kosmetischen Emulsion. Sollten nachfolgend Verhältnisse von Komponenten/Substanzen/Stoffgruppen offenbart werden, so beziehen sich diese Verhältnisse auf Gewichtsverhältnisse der genannten Komponenten/ Substanzen/Stoffgruppen.

Werden nachfolgend Gewichtsprozentbereiche für die Bestandteile der kosmetischen Emulsion angegeben, so umfasst die Offenbarung der vorliegenden Anmeldung ebenfalls alle Einzelwerte in Schritten von 0,1 Gew.-% innerhalb dieser Gewichtsprozentbereiche.

Die Formulierungen "erfindungsgemäß", "erfindungsgemäß vorteilhaft", "vorteilhaft im Sinne der Vorliegenden Erfindung" etc. beziehen sich im Rahmen der vorliegenden Offenbarung immer sowohl auf die erfindungsgemäße Zubereitung sowie die erfindungsgemäße Verwendung.

Sofern nicht anders angegeben wurden alle Versuche unter Normalbedingungen durchgeführt. Der Begriff "Normalbedingungen" bedeutet 20°C, 1013 hPa und eine relative Luftfeuchtigkeit von 50%.

Wird der Begriff Haut verwendet, so bezieht dieser sich bevorzugt auf die menschliche Haut.

Werden in dieser Offenbarung Viskositätswerte angegeben, so beziehen sich alle Werte auf eine Messung bei 25°C in einer 150 ml Weithalsflasche (VWR Nr.: 807-001) mittels Rheomat R 123 von der Firma proRheo. Der Rheomat R 123 der Firma proRheo GmbH ist ein Rotationsviskosimeter, d. h. ein Messkörper rotiert in der zu vermessenden Substanz. Es wird die Kraft gemessen, die benötigt wird, um den Messkörper in der Probe mit einer vorgegebenen Drehzahl rotieren zu lassen. Aus diesem Drehmoment, der Drehzahl des Messkörpers und den geometrischen Abmessungen des verwendeten Messsystems wird die Viskosität berechnet. Als Messkörper wird der Messkörper Nr.2 (Artikelnr. 200 0192), Drehzahl Bereich 62,5 min-1, verwendet.

Erfindungsgemäß weist die Emulsion ein Emulgatorsystem auf, welches in der Emulsion ein flüssigkristallines Netzwerk ausbildet. Die Gegenwart eines derartigen Netzwerks ist entsprechend ein Merkmal der erfindungsgemäßen Emulsion. Es handelt sich vorteilhaft um ein flüssigkristallines Gelnetzwerk.

Es wurde überraschend gefunden, dass das spezifisch in dieser Erfindung ausgebildete Gelnetzwerk in Kombination mit den weiteren Komponenten der Erfindung erlaubt, acrylatfreie, tiegelfähige Emulsionen bereitzustellen, welche nicht aus dem unter Normalbedingungen auslaufen, wenn die Tiegel nach Öffnung gekippt, oder über Kopf gehalten wird. Die Form der Formulierung bleibt erhalten. Dieses ist auch der Fall bei höheren Temperaturen, wie 45°C.

Weiterhin wurde überraschend festgestellt, dass die Konsistenz der Formulierung bei erhöhten Temperaturen sich weniger reduziert.

Das erfindungsgemäße Emulgatorsystem umfasst mindestens ein Fettalkoholglucosid. Vorteilhaft wird das Fettalkoholglucosid ausgewählt aus der Gruppe mit den INCI Bezeichnungen Arachidyl Glucoside; C10-16 Alkyl Glucoside; C12-18 Alkyl Glucoside; C12-20 Alkyl Glucoside; Coco-glucoside; C9-11 Alkyl Glucoside; Caprylyl/Capryl Glucoside; Caprylyl Glucoside; Cetearyl Glucoside; Decyl Glucoside; Isostearyl Glucoside; Lauryl Glucoside; Myristyl Glucoside und/oder Undecyl Glucoside. Bevorzugt sind Cetearyl Glucoside, Caprylyl Glucoside, Caprylyl/Capryl Glucoside, Lauryl Glucoside und Myristyl Glucoside. Insbesondere bevorzugt ist Cetearyl Glucoside.

Vorteilhaft beträgt der Gewichtsanteil von der Gesamtheit der Fettalkoholglucoside in der Emulsion von 0,1 bis 5,0 Gew.-%, bevorzugt von 0,8 bis 3,5 Gew.-%, bevorzugt 1,0 bis 2,5 Gew.-% und insbesondere bevorzugt von 1,2 bis 2,2 Gew.-%, jeweils bezogen auf das Gesamtgewicht der Emulsion.

Es ist ebenfalls vorteilhaft, wenn der Gesamtgewichtsanteil von Arachidyl Glucoside; C10-16 Alkyl Glucoside;

C12-18 Alkyl Glucoside; C12-20 Alkyl Glucoside; Coco-glucoside; C9-11 Alkyl Glucoside; Caprylyl/Capryl Glucoside; Caprylyl Glucoside; Cetearyl Glucoside; Decyl Glucoside; Isostearyl Glucoside; Lauryl Glucoside; Myristyl Glucoside und/oder Undecyl Glucoside von 0,1 bis 5,0 Gew.-%, bevorzugt von 0,8 bis 3,5 Gew.-%, bevorzugt 1,0 bis 2,5 Gew.-% und insbesondere bevorzugt von 1,2 bis 2,2 Gew.-%, jeweils bezogen auf das Gesamtgewicht der Emulsion, beträgt.

Es ist insbesondere vorteilhaft, wenn der Anteil von Cetearyl Glucoside, Caprylyl Glucoside, Caprylyl/Capryl Glucoside, Lauryl Glucoside und/oder Myristyl Glucoside von 0,1 bis 5,0 Gew.-%, bevorzugt von 0,8 bis 3,5 Gew.-%, bevorzugt 1,0 bis 2,5 Gew.-% und insbesondere bevorzugt von 1,2 bis 2,2 Gew.-%, jeweils bezogen auf das Gesamtgewicht der Emulsion, beträgt.

Es ist insbesondere vorteilhaft, wenn der Anteil von Cetearyl Glucoside von 0,1 bis 5,0 Gew.-%, bevorzugt von 0,8 bis 3,5 Gew.-%, bevorzugt 1,0 bis 2,5 Gew.-% und insbesondere bevorzugt von 1,2 bis 2,2 Gew.-%, jeweils bezogen auf das Gesamtgewicht der Emulsion, beträgt.

Weiterhin ist es vorteilhaft, wenn das Emulgatorsystem Sorbitan Stearate umfasst. Sofern Sorbitan Stearate enthalten ist, beträgt der Gesamtanteil von Sorbitan Stearate vorteilhaft von 0,1 bis 1,5 Gew.-%, bevorzugt 0,15 bis 1,0 Gew.-% und insbesondere 0,2 bis 0,8 Gew.-%, bezogen auf das Gesamtgewicht der Emulsion.

Weiterhin ist es vorteilhaft, wenn das Emulgatorsystem Disodium Cetearyl Sulfosuccinate umfasst. Sofern Disodium Cetearyl Sulfosuccinate enthalten ist, beträgt der Gesamtanteil von Disodium Cetearyl Sulfosuccinate vorteilhaft von 0,01 bis 0,5 Gew.-%, bevorzugt 0,02 bis 0,2 Gew.-% und insbesondere 0,04 bis 0,10 Gew.-%, bezogen auf das Gesamtgewicht der Emulsion.

Weiterhin ist es vorteilhaft, wenn das Emulgatorsystem Glyceryl Stearate umfasst. Sofern Glyceryl Stearate enthalten ist, beträgt der Gesamtanteil von Glyceryl Stearate vorteilhaft von 0,1 bis 1,8 Gew.-%, bevorzugt 0,5 bis 1,5 Gew.-% und insbesondere 0,7 bis 1,2 Gew.-%, bezogen auf das Gesamtgewicht der Emulsion.

Weiterhin umfasst das Emulgatorsystem zusätzlich Myristylalkohol. Vorteilhaft beträgt der Anteil des Myristylalkohol von 0,2 bis 6,5 Gew.-%, bevorzugt 0,5 bis 6,0 Gew.-%, bevorzugt 1,5 bis 5,0 Gew.-%, bevorzugt 2,5 bis 4,5 Gew.-% und insbesondere bevorzugt 3,0 bis 4,0 Gew.-%, jeweils bezogen auf das Gesamtgewicht der Emulsion.

Tatsächlich war es überraschend für den Fachmann, dass es möglich ist flüssigkristalline Strukturen mit einem Fettalkohol auszubilden, der einen Schmelzpunkt aufweist, von weniger als 40°C. Üblicherweise ist es nötig Fettalkohole mit einem höheren Schmelzpunkt einzusetzen, wie Stearyl Alkohol. Überraschend erlauben die weiteren Komponenten der Erfindung in Kombination mit Myristylalkohol die Ausbildung der flüssigkristallinen Strukturen.

Flüssigkristalline Strukturen in Emulsionen lassen sich mittels Lichtmikroskopie mit polarisiertem Licht darstellen. Verwiesen wird auf die Veröffentlichung "Study on the Formation and Properties of Liquid Crystal Emulsion in Cosmetic", Wanping Zhang*, Lingyan Liu School of Perfume and Aroma technology, Shanghai Institute of Technology, Shanghai, China.Journal of Cosmetics, Dermatological Sciences and Applications, 2013.

Vorteilhaft liegen die flüssigkristallinen Strukturen in der Emulsion der Erfindung in globularer Struktur vor. Diese ist durch das Auftreten der typischen Malteserkreuze in Lichtmikroskopischen aufnahmen mit polarisiertem Licht erkennbar.

Ferner umfasst die Emulsion der Erfindung mindestens ein Polysaccharid aus den Gruppen der der Stärken und/oder Cellulosen.

Unter den Stärken werden bevorzugt die unter den INCI Bezeichnungen bekannten Substanzen Zea Mays Starch, Sodium Hydroxypropyl Starch Phosphate, Sodium Carboxymethyl Starch, Hydroxypropyl Starch Phosphate, Tapioca Starch, Potato Starch Modified und/oder Distarch Phosphate eingesetzt. Insbesondere bevorzugt ist werden nichtionische Stärken eingesetzt, insbesondere Hydroxypropyl Starch Phosphate und/oder Distarch Phosphate, wobei Hydroxypropyl Starch Phosphate am bevorzugtesten ist.

Unter den Cellulosen werden bevorzugt die unter den INCI Bezeichnungen bekannten Substanzen Ethylcellulose, Microcrystalline Cellulose, Cellulose Gum, Hydroxyethylcellulose, Carboxymethyl Hydroxyethylcellulose, Hydroxyethyl Ethylcellulose, Hydroxymethyl Cellulose, Hydroxypropylcellulose, Hydroxypropyl Methylcellulose und/oder Hydroxyethylcellulose. Vorteilhaft beträgt der Anteil der Polysaccharide in der Emulsion von 0,05 bis 4 Gew.-%, bevorzugt 0,1 bis 2,5 Gew.-%, bevorzugt von 0,2 bis 1,5 Gew.-% und insbesondere bevorzugt von 0,3 bis 1,0 Gew.-%, bezogen auf das Gesamtgewicht der Emulsion. Vorteilhaft beträgt der Anteil der Polysaccharidgummis in der Emulsion von 0,1 bis 4 Gew.-%, bevorzugt 0,2 bis 2,5 Gew.-%, bevorzugt von 0,3 bis 1,5 Gew.-% und insbesondere bevorzugt von 0,4 bis 1,0 Gew.-%, bezogen auf das Gesamtgewicht der Emulsion.

Vorteilhaft beträgt der Anteil der Stärken in der Emulsion von 0,05 bis 4 Gew.-%, bevorzugt 0,1 bis 2,5 Gew.-%, bevorzugt von 0,15 bis 1,5 Gew.-% und insbesondere bevorzugt von 0,2 bis 1,0 Gew.-%, bezogen auf das Gesamtgewicht der Emulsion.

Vorteilhaft beträgt der Gesamtanteil von Zea Mays Starch, Sodium Hydroxypropyl Starch Phosphate, Sodium Carboxymethyl Starch, Hydroxypropyl Starch Phosphate, Tapioca Starch, Potato Starch Modified und/oder Distarch Phosphate in der Emulsion von 0,05 bis 4 Gew.-%, bevorzugt 0,1 bis 2,5 Gew.-%, bevorzugt von 0,15 bis 1,5 Gew.-% und insbesondere bevorzugt von 0,2 bis 1,0 Gew.-%, bezogen auf das Gesamtgewicht der Emulsion.

Vorteilhaft beträgt der Gesamtanteil von Hydroxypropyl Starch Phosphate und/oder Distarch Phosphate in der Emulsion von 0,05 bis 4 Gew.-%, bevorzugt 0,1 bis 2,5 Gew.-%, bevorzugt von 0,15 bis 1,5 Gew.-% und insbesondere bevorzugt von 0,2 bis 1,0 Gew.-%, bezogen auf das Gesamtgewicht der Emulsion.

Vorteilhaft beträgt der Gesamtanteil von Hydroxypropyl Starch Phosphate in der Emulsion von 0,05 bis 4 Gew.-%, bevorzugt 0,1 bis 2,5 Gew.-%, bevorzugt von 0,15 bis 1,5 Gew.-% und insbesondere bevorzugt von 0,2 bis 1,0 Gew.-%, bezogen auf das Gesamtgewicht der Emulsion.

Vorteilhaft beträgt der Anteil der Cellulosen in der Emulsion von 0,05 bis 4 Gew.-%, bevorzugt 0,1 bis 2,5 Gew.-%, bevorzugt von 0,2 bis 1,5 Gew.-% und insbesondere bevorzugt von 0,3 bis 1,0 Gew.-%, bezogen auf das Gesamtgewicht der Emulsion. Vorteilhaft beträgt der Gesamtanteil von Ethylcellulose, Microcrystalline Cellulose, Cellulose Gum, Hydroxyethylcellulose, Carboxymethyl Hydroxyethylcellulose, Hydroxyethyl Ethylcellulose, Hydroxymethyl Cellulose, Hydroxypropylcellulose, Hydroxypropyl Methylcellulose und/oder Hydroxyethylcellulose in der Emulsion von 0,1 bis 4 Gew.-%, bevorzugt 0,05 bis 4 Gew.-%, bevorzugt 0,1 bis 2,5 Gew.-%, bevorzugt von 0,2 bis 1,5 Gew.-% und insbesondere bevorzugt von 0,3 bis 1,0 Gew.-%, bezogen auf das Gesamtgewicht der Emulsion.

Weiterhin umfasst die Emulsion mindestens eine Wachskomponente mit einem Schmelzpunkt von weniger als 43°C.

Vorteilhaft ist mindestens eine Wachskomponente enthalten, welche einen Schmelzpunkt im Bereich von 25°C bis 43°C aufweist. Durch Präsenz derartige niedrigschmelzender Wachskomponenten wird das Schmelzen der Zubereitung auf der menschlichen Haut gewährleistet. Überraschend konnte gleichzeitig die Konsistenz, insbesondere bei Temperaturen um 45°C besser erhalten bleiben (weniger reduziert). Zudem konnte überraschend die Phasentrennungen bei 45°C verhindert werden.

Fettalkohole und Fettsäuren aufweisend Alkylketten mit 10 bis 20 Kohlenstoffatomen zählen definitionsgemäß nicht zu den Wachskomponenten.

Vorteilhaft beträgt der Gesamtgehalt der Wachskomponenten mit einem Schmelzpunkt im Bereich von 25°C bis 43°C von 0,5 bis 5 Gew.-%, bevorzugt 1,5 bis 4,5 Gew.-% und insbesondere bevorzugt 2,2 bis 3,5 Gew.-%, bezogen auf das Gesamtgewicht der Emulsion.

Bevorzugte Wachskomponenten in dem genannten Schmelzbereich sind Myristyl Myristate, Hydrogenated Coco-Glycerides und/oder Cetyl Ricinoleate.

Vorteilhaft beträgt der Gesamtgehalt von Myristyl Myristate, Hydrogenated Coco-Glycerides und/oder Cetyl Ricinoleate von 0,5 bis 5 Gew.-%, bevorzugt 1,5 bis 4,5 Gew.-% und insbesondere bevorzugt 2,2 bis 3,5 Gew.-%, bezogen auf das Gesamtgewicht der Emulsion.

Sofern Myristyl Myristate enthalten ist, ist es vorteilhaft, wenn der Anteil von Myristyl Myristate von 0,5 bis 5,5 Gew.-%, bevorzugt 1,0 bis 4,5 Gew.-% und insbesondere bevorzugt von 1,2 bis 3,0 Gew.-%, bezogen auf das Gesamtgewicht der Emulsion. Sofern Hydrogenated Coco-Glycerides enthalten ist, ist es bevorzugt, wenn der Anteil von Hydrogenated Coco-Glycerides von 0,1 bis 3,0 Gew.-%, bevorzugt 0,3 bis 2,0 Gew.-% und bevorzugt 0,5 bis 1,5 Gew.-%, bezogen auf das Gesamtgewicht der Emulsion. Sofern Cetyl Ricinoleate enthalten ist, ist es bevorzugt, wenn der Anteil von Cetyl Ricinoleate von 0,01 bis 3,0 Gew.-%, bevorzugt 0,05 bis 1,0 Gew.-% und bevorzugt 0,1 bis 0,5 Gew.-%, bezogen auf das Gesamtgewicht der Emulsion.

Weiterhin ist erfindungsgemäß von Vorteil, wenn hochschmelzende Wachse mit einem Schmelzpunkt von mehr als 43°C nicht enthalten sind oder deren Anteile weniger als 0,5 Gew.-% der Emulsion betragen. Überraschend konnten bei 45°C stabile Formulierungen bereitgestellt werden, ohne dass hochschmelzende Wachskomponenten eingearbeitet werden müssen. Es wird vermutet, dass die Ausbildung der flüssigkristallinen Strukturen bei 45°C die Stabilität der Formulierung zur Verhinderung von Phasentrennungen zum Erhalt der Konsistenz ermöglicht.

Weiterhin ist es vorteilhaft, wenn die Emulsion ein oder mehrere unter Normalbedingungen flüssige Ölkomponenten umfasst. Generell ist es bevorzugt, wenn der Anteil der unter Normalbedingungen flüssige Ölkomponenten von 0,2 bis 5,0 Gew.-%, bevorzugt 0,5 bis 4.0 Gew.-% und insbesondere bevorzugt 1,5 bis 2,5 Gew.-%, bezogen auf das Gesamtgewicht der Emulsion.

Vorteilhaft enthaltene Öle sind gewählt aus natürlichen Ölen. Vorteilhaft enthaltene natürliche Öle sind gewählt aus der Gruppe Persea Gratissima Oil, Orbignya Oleifera Seed Oil, Argania Spinosa Kernel Oil, Prunus Armeniaca Kernel Oil, Simmondsia Chinensis Seed Oil, Butyrospermum Parkii Butter, Cocos Nucifera Oil, Silybum Marianum Seed Oil, Oenothera Biennis Oil, Olea Europaea Fruit Oil, Helianthus Annuus Seed Oil, Vitis Vinifera Seed Oil, Cannabis Sativa Seed Oil, Vegetable Oil, Gossypium Herbaceum Seed Oil, Arctium Lappa Seed Oil, Macadamia Ternifolia Seed Oil, Macadamia Integrifolia Seed Oil, Zea Mays Germ Oil, Prunus Amygdalus Dulcis Oil, Ricinus Communis Seed Oil, Brassica Campestris Seed Oil und/oder Glycine Soja Oil. Sofern natürliche Öle enthalten sind, beträgt der Gesamtgehalt dieser Öle vorteilhaft von 0,01 bis 3 Gew.-%, bevorzugt 0,1 bis 1,0 Gew.-% und insbesondere bevorzugt von 0,2 bis 0,8 Gew.-%, bezogen auf das Gesamtgewicht der Emulsion.

Weiterhin ist es vorteilhaft, wenn ein oder mehrere Ester des Kokosfettalkohols enthalten sind. Bevorzugt wird hier insbesondere Coco-Caprylate/Caprate eingesetzt. Sofern ein Ester eines Kokosfettalkohols enthalten ist, beträgt der Anteil dieser Ester vorteilhaft von 0,2 bis 4 Gew.-%, bevorzugt 0,4 bis 2,5 Gew.-% und insbesondere bevorzugt von 0,8 bis 1,5 Gew.-%, bezogen auf das Gesamtgewicht der Emulsion. Sofern Coco-Caprylate/Caprate enthalten ist, beträgt der Anteil von Coco-Caprylate/Caprate vorteilhaft von 0,2 bis 4 Gew.-%, bevorzugt 0,4 bis 2,5 Gew.-% und insbesondere bevorzugt von 0,8 bis 1,5 Gew.-%, bezogen auf das Gesamtgewicht der Emulsion.

Weiterhin ist es bevorzugt, wenn ein oder mehrere Öle enthalten sind, die Triglyceride von 3 identischen Fettsäuren sind. Insbesondere bevorzugt ist Triisostearin. Sofern Triglyceride von 3 identischen Fettsäuren enthalten sind, beträgt der Anteil dieser Triglyceride vorteilhaft von 0,05 bis 2 Gew.-%, bevorzugt 0,08 bis 1,0 Gew.-% und insbesondere bevorzugt von 0,1 bis 0,5 Gew.-%, bezogen auf das Gesamtgewicht der Emulsion. Sofern Triisostearin enthalten ist, beträgt der Anteil von Triisostearin vorteilhaft von 0,05 bis 2 Gew.-%, bevorzugt 0,08 bis 1,0 Gew.-% und insbesondere bevorzugt von 0,1 bis 0,5 Gew.-%, bezogen auf das Gesamtgewicht der Emulsion.

Zusätzlich ist es vorteilhaft, wenn ein oder mehrere Fettalkohole aufweisend 16 bis 24 Kohlenstoffatome enthalten sind, wobei der Gesamtanteil dieser Fettalkohole von 0,01 bis 1,5 Gew.-%, bevorzugt von 0,05 bis 0,3 Gew.-% beträgt, bezogen auf das Gesamtgewicht der Emulsion. Insbesondere ist Cetearylalkohol in einem Anteil von von 0,01 bis 1,0 Gew.-%, bevorzugt von 0,05 bis 0,3 Gew.-%bezogen auf das Gesamtgewicht der Emulsion enthalten.

Weiterhin hat sich gezeigt, dass es vorteilhaft ist, wenn ein oder mehrere Fettsäuren aufweisend 14 bis 24 Kohlenstoffatome in der Emulsion enthalten sind, wobei deren Anteil vorteilhaft von 0,2 bis 5,0 Gew.-%, bevorzugt von 1,5 Gew.-% bis 4 Gew.-% und insbesondere bevorzugt von 1,8 bis 3,0 Gew.-%, bezogen auf das Gesamtgewicht der Emulsion beträgt.

Ferner ist die erfindungsgemäße Emulsion bevorzugt dadurch gekennzeichnet, dass diese Emulison Glycerin in einem Anteil von 0,5 Gew.-% bis 15% Gew.-%, bevorzugt 3,0 bis 7 Gew.-%, bezogen auf das Gesamtgewicht der Emulsion, enthält.

Weiterhin ist es ebenfalls vorteilhaft im Sinne der vorliegenden Erfindung, wenn die Emulsion Phenoxyethanol, Dehydracetsäure, Benzyl Alkohol und/oder Ethylhexylglycerin enthält.

Enthält die Emulsion Benzylalkohol so ist es bevorzugt, wenn der Anteil an Benzylalkohol von 0,05 Gew.-% bis 0,5 Gew.-% beträgt, bezogen auf das Gesamtgewicht der Emulsion.

Enthält die Emulsion Phenoxyethanol, so ist es bevorzugt, wenn der Gesamtanteil an Phenoxyethanol von 0,1 Gew.-% bis 1,2 Gew.-% beträgt, bezogen auf das Gesamtgewicht der Emulsion.

Enthält die Emulsion Ethylhexylglycerin, so ist es bevorzugt, wenn der Anteil an Ethylhexylglycerin von 0,1 Gew.-% bis 1,0 Gew.-% beträgt, bezogen auf das Gesamtgewicht der Emulsion.

Darüber hinaus ist es vorteilhaft, wenn Ausführungsformen der Erfindung dadurch gekennzeichnet sind, dass diese Ethanol enthalten. Ist Ethanol in der Emulsion enthalten, so beträgt der Anteil von Ethanol bevorzugt von 0,5 Gew.-% bis 10 Gew.-%, bevorzugt 1,5 Gew.-% bis 5,0 Gew.-% bezogen auf das Gesamtgewicht der Emulsion.

Nicht zuletzt sind erfindungsgemäß vorteilhafte Ausführungsformen dadurch gekennzeichnet, dass die Emulsion einen oder mehrere Wirkstoffe gewählt aus der Gruppe der Verbindungen Glycyrrhetinsäure, Harnstoff, Arctiin, Folsäure, Coenzym Q10 (Ubiquinon), alpha-Glucosylrutin, Carnitin, Camosin, Coffein, natürliche und/oder synthetische Isoflavonoide, Glycerylglucose, Kreatin, Kreatinin, Taurin, Tocopherol, Tocopherolacetat, Vitamin C, Vitamin C phosphate, Vitamin C palmitate, Niacinamide, Vitamin A palmitate, Panthenol, Licochalcon A, Rucinol, N-[(2,4-Dihydroxyphenyl)thiazol-2-yl]isobutyramide, Honociol und Magnolol (auch als Bestandteil von Magnolia-Extrakten), Hyaluronsäure und/oder Silymarin (Mariendistelextrakt) enthält.

Ferner sind erfindungsgemäß vorteilhafte Emulsionen dadurch gekennzeichnet, dass diese Wasser in einem Anteil von 60 Gew.-% bis 95 Gew.-% und bevorzugt von 70 Gew.-% bis 90 Gew.-%, bezogen auf das Gesamtgewicht der Emulsion enthalten.

Weiterhin weisen erfindungsgemäß vorteilhafte Emulsionen 24 h nach Herstellung eine Viskosität von 9000 mPa·s bis 30000 mPa·s, vorteilhaft 12000 mPa·s bis 20000 mPa·s auf. Wird in dieser Offenbarung von Viskosität gesprochen, so beziehen sich alle Werte auf eine Messung bei 25°C im 150 ml Rollrandglas mittels Rheomat R 123 von der Firma proRheo. Der Rheomat R 123 der Firma proRheo GmbH ist ein Rotationsviskosimeter, d. h. ein Messkörper rotiert in der zu vermessenden Substanz. Es wird die Kraft gemessen, die benötigt wird, um den Messkörper in der Probe mit einer vorgegebenen Drehzahl rotieren zu lassen. Aus diesem Drehmoment, der Drehzahl des Messkörpers und den geometrischen Abmessungen des verwendeten Messystems wird die Viskosität berechnet. Als Messkörper wird der Messkörper Nr.2 (Artikelnr. 200 0192), Drehzahl Bereich 62,5 min$^{-1}$, verwendet. Alle Messungen zur Viskosität erfolgen immer 24h nach Herstellung der Emulsion.

## Vergleichsversuche und Beispiele

Die nachfolgenden Beispiele sollen die vorliegende Erfindung verdeutlichen, ohne sie einzuschränken. Alle Mengenangaben, Anteile und Prozentanteile sind, soweit nicht anders angegeben, auf das Gewicht und die Gesamtmenge bzw. auf das Gesamtgewicht der Zubereitungen bezogen.

Die nachfolgende Tabelle mit den Bsp.1 bis Bsp. 8 zeigt verschiedene Formulierungen. Bsp.1 ist ein nicht erfindungsgemäßes Beispiel, welches eine übliche Formulierung mit dem Acrylat-basierten Polymer Carbomer darstellt. Die Formulierung weist keine flüssigkristallinen Strukturen auf. Die Formulierung ist sowohl stabil gegen Wasserabscheidungen bei Lagerung bei 40 und 45°C über 8 Monate. Zudem weist diese Formulierung auch bei hohen Temperaturen von 45°C eine ansprechende Konsistenz auf.

Wie die nicht erfindungsgemäßen Beispiele 2 bis 4 zeigen, führt der Austausch von acrylatbasierten Polymeren mit einem Biopolymer dazu, dass die Formulierungen eine unzureichende Stabilität gegen Wasserabscheidungen aufweisen und auch eine geringe Konsistenz bei 45°C aufweisen. Diese Beispiele zeigen keine flüssigkristallinen Strukturen.

Die Beispiele 5 bis 7 sind erfindungsgemäße Beispiele. Diese weisen flüssigkristalline Strukturen auf, welche mittels Lichtmikroskopie mit polarisiertem Licht nachgewiesen wurden. Überraschend wurde gefunden, dass die Gegenwart der flüssigkristallinen Strukturen zu einer Stabilisierung der Formulierungen führt, so dass Wasserabscheidungen bei erhöhten Temperaturen bei 8-monatiger Lagerung verhindert werden. Zudem konnte überraschend eine deutlich erhöhte Konsistenz bei 45°C ermittelt werden. Folglich wurden die durch den Verzicht auf acrylathaltige Polymere entstanden Nachteile durch die vorliegende Erfindung abgemildert.

Die Messung der Konsistenz erfolgte mittels des in DE 2909087 C2 beschriebenen Verfahrens.

Eine 8 mm Polyamidkugel als Messinstrument wird einmal unter definierten Bedingungen (mit gleichmäßiger Geschwindigkeit und konstanter maximaler Eintauchtiefe) entlang einer Rundstrecke durch die Prüfemulsion gezogen. Die von der Cremekonsistenz abhängige Bremskraft wird mit einem Blattfederprüfstand gemessen, an dessen Fühler die Kugel befestigt ist. Da die Kugel nur einmal durch jede Zone der Creme gezogen wird, werden Einflussfaktoren, beispielsweise durch thixotrope Eigenschaften und mögliche Gleit-Fließ-Effekte, weitgehend vermieden. Die Drehzahl der Rotationsachse wird auf 10 U/min eingestellt. Dabei wird die auf die Kugel wirkende maximale Bremskraft ermittelt. Entscheidend ist, dass bei allen Messungen die Kugel gleich tief und weit eintaucht. Die ermittelten Werte sind Skalenwerte, wobei ein Skalenwert eine 1 cN entspricht. Die Skalenwerte sind in der Tabelle mit den Formulierungen angegeben. Die Proben Temperatur betrug 45°C.

Weiterhin wurde eine optische Bewertung wie in der nachfolgenden Tabelle angegeben für die Beispiele vorgenommen. Weiterhin wurde das Speichermodul G' (Storage Module G') der Formulierungen Bsp.1, Bsp.2 und Bsp.6 ermittelt. Der Speichermodul gibt Auskunft über die Menge an Struktur, die in einem Material vorhanden ist. Es stellt die Energie dar, die in der elastischen Struktur der Probe gespeichert ist.

Es wurden die folgenden Messparameter gewählt:

| Instrument: | ARES 6 |
| Methode: | ERW (Erweichen, soften, DynamicTemperatureRamp) |
| Temperatur: | 20°C bis 80°C mit 2°C/min |
| Geometrie: | Parallele Platten 50 mm und 1,0mm Spalt |
| Frequenz: | 100 rad s-1 |

Die Messergebnisse bei 20°C und 45°C sind in der folgenden Tabelle aufgeführt:

| Samples | Storage Module [Pa] @20°C | Storage Module [Pa] @45°C | $\frac{G'@45°C}{G'@20°C} \times 100\%$ |
|---|---|---|---|
| Bsp.1 | 2900 | 1000 | 34% |
| Bsp.2 | 1700 | 400 | 23% |
| Bsp.6 | 2000 | 800 | 40% |

Vergleicht man die Storage Module bei 20°C und 45°C für die jeweiligen Proben fällt auf, dass Bsp.6 bei 45 °C immer noch eine ähnliche Menge (ca. 40 %) ihrer Speichermodule vim Vergleich zum Wert bei 20°C aufweist. Dieses ist vergleichbar mit den Werten von Bsp .1, welches ein Acrylat-haltiges Polymer enthält. Bsp.2 kann bei 45°C kann nicht die gleiche Werte wie Bsp.1 halten.

| INCI | | Bsp.1 | Bsp.2 | Bsp.3 | Bsp.4 | Bsp.5 | Bsp.6 | Bsp.7 |
|---|---|---|---|---|---|---|---|---|
| Vegetable Oil | | 0,3 | 0,3 | 0,3 | 0,3 | 0,3 | 0,3 | 0,3 |
| Coco-Caprylate/Caprate | | 1,2 | 1,2 | 1,2 | 1,2 | 1,2 | 1,2 | 1,2 |

(fortgesetzt)

| INCI | | Bsp.1 | Bsp.2 | Bsp.3 | Bsp.4 | Bsp.5 | Bsp.6 | Bsp.7 |
|---|---|---|---|---|---|---|---|---|
| Simmondsia Chinensis Seed Oil | | 0,1 | 0,1 | 0,1 | 0,1 | 0,1 | 0,1 | 0,1 |
| Prunus Amygdalus Dulcis Oil | | 0,1 | 0,1 | 0,1 | 0,1 | 0,1 | 0,1 | 0,1 |
| Triisostearin | | 0,3 | 0,3 | 0,3 | 0,3 | 0,3 | 0,3 | 0,3 |
| Myristyl Alcohol | | 3,5 | 3,5 | 3,5 | 2,5 | 3,5 | 3,5 | 3,5 |
| Hydrogenated Coco-Glycerides | | 1 | 1 | 1 | 1 | 1 | 1 | 1 |
| Myristyl Myristate | | 1,5 | 1,5 | 1,5 | 1,5 | 1,5 | 1,5 | 1,5 |
| Cetyl Ricinoleate | | 0,1 | 0,1 | 0,1 | 0,1 | 0,1 | 0,1 | 0,1 |
| Cetearyl Alcohol | | 0,1 | 0,1 | 0,1 | 0,1 | 0,1 | 0,1 | 0,1 |
| Glyceryl Stearate | | 1,1 | 1,1 | 1,1 | 1,1 | 1,1 | 1,1 | 1,1 |
| Palmitic Acid | | 1,2 | 1,2 | 1,2 | 1,2 | 1,2 | 1,2 | 1,2 |
| Stearic Acid | | 1,2 | 1,2 | 1,2 | 1,2 | 1,2 | 1,2 | 1,2 |
| Glyceryl Stearate Citrate | | 1,5 | 1,5 | 1,5 | 0,1 | | | |
| Polyglyceryl-3 Methylglucose Distearate | | | | 0,8 | | | | |
| Polyglyceryl-3 Distearate | | | | | 2,5 | | | |
| Cetearyl Glucoside | | | | | | 1,8 | 1,5 | 1,0 |
| Sorbitan Stearate | | | | | | 0,8 | 0,7 | 0,4 |
| Disodium Cetearyl Sulfosuccinate | | | | | | 0,1 | 0,1 | 0,1 |
| Hydroxypropyl Starch Phosphate | | 0 | 0,8 | 0,8 | 0,8 | 0,8 | 0,8 | 0,8 |
| Carbomer | | 0,3 | 0 | 0 | 0 | 0 | 0 | 0 |
| Parfum | | 0,2 | 0,2 | 0,2 | 0,2 | 0,2 | 0,2 | 0,2 |
| Glycerin | | 4,5 | 4,5 | 4,5 | 4,5 | 4,5 | 4,5 | 4,5 |
| Sodium Hydroxide | | q.s. | q.s. | q.s. | q.s. | q.s. | q.s. | q.s. |
| Phenoxyethanol | | 0,9 | 0,9 | 0,9 | 0,9 | 0,9 | 0,9 | 0,9 |
| Alcohol Denat. | | 3 | 3 | 3 | 3 | 3 | 3 | 3 |
| Aqua | | Ad. 100 | Ad. 100 | Ad. 100 | Ad. 100 | Ad. 100 | Ad. 100 | Ad. 100 |
| Stabilität 8 Monate (Wasserabscheidung, 100%=keine Wasserabscheidung) | 40°C | 100 | 20 | 40 | 60 | 100 | 100 | 100 |
| | 45°C | 100 | 0 | 20 | 20 | 100 | 100 | 100 |
| Konsistenz Skalenwert bei 45°C | | 14 | 2 | 2 | 3 | 12 | 11 | 7 |

| Optische Bewertung im Vergleich von 45°C Proben zu Proben bei 20°C (visuelle Beurteilung der Cremebewegung in einer 30 ml Glasflasche, wenn die Flasche überkopf gestellt wird) | Keine Bewegung der Formel | Formel läuft sofort nach unten, Verhalten wie Wasser | Formel läuft sofort nach unten, Verhalten wie Wasser | Formel läuft sofort nach unten, Verhalten wie Wasser | Keine Bewegung der Formel | Keine Bewegung der Formel | Keine Bewegung der Formel |

Weitere Beispiele:

| INCI | Bsp A | Bsp. B | Bsp. C | Bsp. D | Bsp. E | Bsp. F |
|---|---|---|---|---|---|---|
| Tocopherol | 0,1 | 0 | 0 | 0,2 | 0 | 0 |
| Vegetable Oil | 0 | 0,3 | 0 | 0 | 0 | 0,4 |
| Coco-Caprylate/Caprate | 0 | 0 | 1 | 0 | 0 | 0 |
| Ethylhexyl Cocoate | 1,0 | 0 | 0 | 0 | 0 | 0 |
| Octyldodecanol | 0 | 1 | 0 | 0 | 0,6 | 0 |
| Simmondsia Chinensis Seed Oil | 0 | 0 | 0,1 | 0 | 0 | 0 |
| Butyrospermum Parkii Butter | 0,1 | 0,5 | 0 | 0,4 | 0,7 | 0 |
| Glycine Soja Oil | 0 | 0 | 0,5 | 0 | 0 | 0 |
| Prunus Amygdalus Dulcis Oil | 0 | 0 | 0 | 0 | 0 | 0,2 |
| Helianthus Annuus Seed Oil | 0 | 0 | 0,1 | 1,5 | 0 | 0,5 |
| Brassica Campestris Seed Oil | 0 | 0 | 0 | 0 | 0,2 | 0 |
| Triisostearin | 0 | 0,1 | 0 | 0 | 0,3 | 0 |
| Myristyl Alcohol | 5 | 4 | 3 | 3 | 5 | 5 |
| Hydrogenated Coco-Glycerides | 0,7 | 0,6 | 1 | 1,5 | 1,3 | 1 |
| Myristyl Myristate | 1 | 1,2 | 1,5 | 1 | 0,8 | 0,7 |
| Cetyl Ricinoleate | 0,1 | 0,1 | 0,3 | 0,2 | 0,1 | 0,4 |
| Cetearyl Alcohol | 0,2 | 1 | 0,1 | 1,5 | 0,1 | 0,2 |
| Cetyl Palmitate | 0 | 0 | 0 | 0,2 | 0 | 0 |
| Glyceryl Stearate | 1,3 | 0,7 | 1,2 | 0,8 | 1,1 | 0,7 |
| Palmitic Acid | 1,5 | 1 | 1,3 | 0,8 | 1,1 | 1 |
| Stearic Acid | 1,5 | 1 | 1,3 | 0,8 | 1,1 | 1 |
| Cetearyl Glucoside | 0,9 | 1 | 1,6 | 1,5 | 0,7 | 2,2 |
| Sorbitan Stearate | 0,2 | 0,3 | 0,7 | 0,1 | 0,5 | 1,2 |
| Disodium Cetearyl Sulfosuccinate | 0,1 | 0 | 0,1 | 0 | 0 | 0 |
| Hydroxypropyl Starch Phosphate | 0,5 | 0,4 | 0,6 | 0 | 0,8 | 0 |
| Distarch Phosphate | 0 | 0,4 | 0 | 1 | 0 | 2 |
| Gellan Gum | 0,2 | 0 | 0 | 0,1 | 0,1 | 0 |

(fortgesetzt)

| INCI | Bsp A | Bsp. B | Bsp. C | Bsp. D | Bsp. E | Bsp. F |
|---|---|---|---|---|---|---|
| Xanthan Gum | 0 | 0 | 0,1 | 0 | 0 | 0 |
| Parfum | 0,1 | 0,3 | 0,3 | 0,2 | 0,6 | 0,4 |
| Glycerin | 3 | 5 | 3,5 | 10 | 4 | 7 |
| Sodium Hydroxide | q.s. | q.s. | q.s. | q.s. | q.s. | q.s. |
| Phenoxyethanol | 0,7 | 0,9 | 0 | 0,8 | 0,3 | 0,4 |
| Ethylhexylglycerin | 0,1 | 0 | 0,3 | 0 | 0,2 | 0,2 |
| Hydroxyacetophenone | 0 | 0 | 0,1 | 0 | 0 | 0,2 |
| Benzyl alcohol | 0 | 0 | 0,3 | 0,1 | 0,1 | 0 |
| Alcohol Denat. | 3 | 4 | 5 | 3 | 5 | 5 |
| Aqua | Ad 100 | Ad 100 | Ad 100 | Ad 100 | Ad 100 | Ad 100 |

**Patentansprüche**

1.  Acrylatfreie kosmetische Emulsion aufweisend

    a) Ein Emulgatorsystem, welches in der Emulsion ein flüssigkristallines Netzwerk ausbildet, wobei das Emulgatorsystem

        a. Mindestens ein Fettalkoholglucosid, und
        b. Myristylalkohol

    umfasst,
    b) Mindestens ein Polysaccharid, und
    c) Weiterhin mindestens eine Wachskomponente mit einem Schmelzpunkt von weniger als 43°C

    **dadurch gekennzeichnet, dass**

    - die Polysaccharide aus den Gruppen der Stärken und/oder Cellulosen gewählt werden und
    - Fettalkohole und Fettsäuren aufweisend Alkylketten mit 10 bis 20 Kohlenstoffatomen definitionsgemäß nicht zu den Wachskomponenten zählen.

2.  Emulsion nach Anspruch 1 **dadurch gekennzeichnet, dass** es sich um eine Öl-in-Wasser Emulsion handelt.

3.  Emulsion nach einem der vorhergehenden Ansprüche **dadurch gekennzeichnet, dass** das Fettalkoholglucosid ausgewählt aus der Gruppe mit den INCI Bezeichnungen Arachidyl Glucoside; C10-16 Alkyl Glucoside; C12-18 Alkyl Glucoside; C12-20 Alkyl Glucoside; Coco-glucoside; C9-11 Alkyl Glucoside; Caprylyl/Capryl Glucoside; Caprylyl Glucoside; Cetearyl Glucoside; Decyl Glucoside; Isostearyl Glucoside; Lauryl Glucoside; Myristyl Glucoside und/oder Undecyl Glucoside.

4.  Emulsion nach Anspruch 3 **dadurch gekennzeichnet, dass** Cetearyl Glucoside, Caprylyl Glucoside, Caprylyl/Capryl Glucoside, Lauryl Glucoside und Myristyl Glucoside enthalten sind, wobei Cetearyl Glucoside insbesondere bevorzugt ist.

5.  Emulsion nach einem der vorhergehenden Ansprüche **dadurch gekennzeichnet, dass** der Gewichtsanteil von der Gesamtheit der Fettalkoholglucoside in der Emulsion von 0,1 bis 5,0 Gew.-%, bevorzugt von 0,8 bis 3,5 Gew.-%, bevorzugt 1,0 bis 2,5 Gew.-% und insbesondere bevorzugt von 1,2 bis 2,2 Gew.-%, jeweils bezogen auf das Gesamtgewicht der Emulsion, beträgt.

6.  Emulsion nach einem der Ansprüche 3 bis 5 **dadurch gekennzeichnet, dass** der Anteil von Cetearyl Glucoside von 0,1 bis 5,0 Gew.-%, bevorzugt von 0,8 bis 3,5 Gew.-%, bevorzugt 1,0 bis 2,5 Gew.-% und insbesondere bevorzugt von 1,2 bis 2,2 Gew.-%, jeweils bezogen auf das Gesamtgewicht der Emulsion, beträgt.

7. Emulsion nach einem der vorhergehenden Ansprüche **dadurch gekennzeichnet, dass** das Emulgatorsystem Sorbitan Stearate umfasst, wobei der Gesamtanteil von Sorbitan Stearate vorteilhaft von 0,1 bis 1,5 Gew.-%, bevorzugt 0,15 bis 1,0 Gew.-% und insbesondere 0,2 bis 0,8 Gew.-%, bezogen auf das Gesamtgewicht der Emulsion, beträgt.

8. Emulsion nach einem der vorhergehenden Ansprüche **dadurch gekennzeichnet, dass** das Emulgatorsystem Disodium Cetearyl Sulfosuccinate umfasst, wobei es vorteilhaft ist, wenn der Gesamtanteil von Disodium Cetearyl Sulfosuccinate von 0,01 bis 0,5 Gew.-%, bevorzugt 0,02 bis 0,2 Gew.-% und insbesondere 0,04 bis 0,10 Gew.-%, bezogen auf das Gesamtgewicht der Emulsion, beträgt.

9. Emulsion nach einem der vorhergehenden Ansprüche **dadurch gekennzeichnet, dass** das Emulgatorsystem Glyceryl Stearate umfasst, wobei es vorteilhaft ist, wenn der Gesamtanteil von Glyceryl Stearate von 0,1 bis 1,8 Gew.-%, bevorzugt 0,5 bis 1,5 Gew.-% und insbesondere 0,7 bis 1,2 Gew.-%, bezogen auf das Gesamtgewicht der Emulsion, beträgt.

10. Emulsion nach einem der vorhergehenden Ansprüche **dadurch gekennzeichnet, dass** der Anteil des Myristylalkohol von 0,2 bis 6,5 Gew.-%, bevorzugt 0,5 bis 6,0 Gew.-%, bevorzugt 1,5 bis 5,0 Gew.-%, bevorzugt 2,5 bis 4,5 Gew.-% und insbesondere bevorzugt 3,0 bis 4,0 Gew.-%, jeweils bezogen auf das Gesamtgewicht der Emulsion, beträgt.

11. Emulsion nach einem der vorhergehenden Ansprüche **dadurch gekennzeichnet, dass** die flüssigkristallinen Strukturen in der Emulsion in globularer Struktur vorliegen.

12. Emulsion nach einem der vorhergehenden Ansprüche **dadurch gekennzeichnet, dass** als Polysaccharide Zea Mays Starch, Sodium Hydroxypropyl Starch Phosphate, Sodium Carboxymethyl Starch, Hydroxypropyl Starch Phosphate, Tapioca Starch, Potato Starch Modified und/oder Distarch Phosphate enthalten sind, wobei Hydroxypropyl Starch Phosphate und Distarch Phosphat bevorzugt sind, insbesondere bevorzugt ist Hydroxypropyl Starch Phosphate.

13. Emulsion nach einem der vorhergehenden Ansprüche **dadurch gekennzeichnet, dass** der Anteil der Stärken in der Emulsion von 0,05 bis 4 Gew.-%, bevorzugt 0,1 bis 2,5 Gew.-%, bevorzugt von 0,15 bis 1,5 Gew.-% und insbesondere bevorzugt von 0,2 bis 1,0 Gew.-%, bezogen auf das Gesamtgewicht der Emulsion, beträgt.

14. Emulsion nach einem der einem Ansprüche **dadurch gekennzeichnet, dass** der Gesamtgehalt der Wachskomponenten mit einem Schmelzpunkt im Bereich von 25°C bis 43°C von 0,5 bis 5 Gew.-%, bevorzugt 1,5 bis 4,5 Gew.-% und insbesondere bevorzugt 2,2 bis 3,5 Gew.-%, bezogen auf das Gesamtgewicht der Emulsion, beträgt.

## Claims

1. Acrylate-free cosmetic emulsion comprising

    a) an emulsifier system which forms a liquid-crystalline network in the emulsion, the emulsifier system comprising

        a. at least one fatty alcohol glucoside, and
        b. myristyl alcohol,

    b) at least one polysaccharide, and
    c) furthermore, at least one wax component having a melting point of less than 43°C,

    **characterized in that**

        - the polysaccharides are selected from the groups of the starches and/or celluloses and
        - fatty alcohols and fatty acids having alkyl chains with 10 to 20 carbon atoms by definition are not considered to be wax components.

2. Emulsion according to Claim 1, **characterized in that** it is an oil-in-water emulsion.

3. Emulsion according to either of the preceding claims, **characterized in that** the fatty alcohol glucoside is selected from the group having the INCI names Arachidyl Glucoside; C10-16 Alkyl Glucoside; C12-18 Alkyl Glucoside; C12-20 Alkyl Glucoside; Coco-glucoside; C9-11 Alkyl Glucoside; Caprylyl/Capryl Glucoside; Caprylyl Glucoside; Cetearyl Glucoside; Decyl Glucoside; Isostearyl Glucoside; Lauryl Glucoside; Myristyl Glucoside and/or Undecyl Glucoside.

4. Emulsion according to Claim 3, **characterized in that** Cetearyl Glucoside, Caprylyl Glucoside, Caprylyl/Capryl Glucoside, Lauryl Glucoside and Myristyl Glucoside are present, with Cetearyl Glucoside being especially preferred.

5. Emulsion according to any of the preceding claims, **characterized in that** the proportion by weight of the entirety of the fatty alcohol glucosides in the emulsion is from 0.1% to 5.0% by weight, preferably from 0.8% to 3.5% by weight, preferably 1.0% to 2.5% by weight and especially preferably from 1.2% to 2.2% by weight, in each case based on the total weight of the emulsion.

6. Emulsion according to any of Claims 3 to 5, **characterized in that** the proportion of Cetearyl Glucoside is from 0.1% to 5.0% by weight, preferably from 0.8% to 3.5% by weight, preferably 1.0% to 2.5% by weight and especially preferably from 1.2% to 2.2% by weight, in each case based on the total weight of the emulsion.

7. Emulsion according to any of the preceding claims, **characterized in that** the emulsifier system comprises Sorbitan Stearate, with the total proportion of Sorbitan Stearate advantageously being from 0.1% to 1.5% by weight, preferably 0.15% to 1.0% by weight and in particular 0.2% to 0.8% by weight, based on the total weight of the emulsion.

8. Emulsion according to any of the preceding claims, **characterized in that** the emulsifier system comprises Disodium Cetearyl Sulfosuccinate, with it being advantageous if the total proportion of Disodium Cetearyl Sulfosuccinate is from 0.01% to 0.5% by weight, preferably 0.02% to 0.2% by weight and in particular 0.04% to 0.10% by weight, based on the total weight of the emulsion.

9. Emulsion according to any of the preceding claims, **characterized in that** the emulsifier system comprises Glyceryl Stearate, with it being advantageous if the total proportion of Glyceryl Stearate is from 0.1% to 1.8% by weight, preferably 0.5% to 1.5% by weight and in particular 0.7% to 1.2% by weight, based on the total weight of the emulsion.

10. Emulsion according to any of the preceding claims, **characterized in that** the proportion of the myristyl alcohol is from 0.2% to 6.5% by weight, preferably 0.5% to 6.0% by weight, preferably 1.5% to 5.0% by weight, preferably 2.5% to 4.5% by weight and especially preferably 3.0% to 4.0% by weight, in each case based on the total weight of the emulsion.

11. Emulsion according to any of the preceding claims, **characterized in that** the liquid-crystalline structures in the emulsion are present in globular structure.

12. Emulsion according to any of the preceding claims, **characterized in that** the polysaccharides present are Zea Mays Starch, Sodium Hydroxypropyl Starch Phosphate, Sodium Carboxymethyl Starch, Hydroxypropyl Starch Phosphate, Tapioca Starch, Potato Starch Modified and/or Distarch Phosphate, with Hydroxypropyl Starch Phosphate and Distarch Phosphate being preferred, Hydroxypropyl Starch Phosphate being especially preferred.

13. Emulsion according to any of the preceding claims, **characterized in that** the proportion of the starches in the emulsion is from 0.05% to 4% by weight, preferably 0.1% to 2.5% by weight, preferably from 0.15% to 1.5% by weight and especially preferably from 0.2% to 1.0% by weight, based on the total weight of the emulsion.

14. Emulsion according to any of the any claims, **characterized in that** the total content of the wax component having a melting point in the range from 25°C to 43°C is from 0.5% to 5% by weight, preferably from 1.5% to 4.5% by weight and especially preferably 2.2% to 3.5% by weight, based on the total weight of the emulsion.

**Revendications**

1. Émulsion cosmétique exempte d'acrylates, comprenant

a) un système émulsifiant, qui forme dans l'émulsion un réseau liquide cristallin, le système émulsifiant comprenant

a. au moins un glucoside d'alcool gras, et
b. de l'alcool myristylique,

b) au moins un polysaccharide, et
c) en outre au moins un composant cireux ayant un point de fusion inférieur à 43 °C,

**caractérisée en ce que**

- les polysaccharides sont choisis dans les groupes des amidons et/ou des celluloses et
- les alcools gras et les acides gras ayant des chaînes alkyles ayant de 10 à 20 atomes de carbone ne sont pas, par définition, inclus dans les composants cireux.

**2.** Émulsion selon la revendication 1, **caractérisée en ce qu'**il s'agit d'une émulsion huile-dans-eau.

**3.** Émulsion selon l'une quelconque des revendications précédentes, **caractérisée en ce que** le glucoside d'alcool gras est choisi dans le groupe comprenant les désignations INCI Arachidyl Glucoside ; C10-16 Alkyl Glucoside ; C12-18 Alkyl Glucoside ; C12-20 Alkyl Glucoside ; Coco-glucoside ; C9-11 Alkyl Glucoside ; Caprylyl/Capryl Glucoside ; Caprylyl Glucoside ; Cetearyl Glucoside ; Decyl Glucoside ; Isostearyl Glucoside ; Lauryl Glucoside ; Myristyl Glucoside et/ou Undecyl Glucoside.

**4.** Émulsion selon la revendication 3, **caractérisée en ce que** Cetearyl Glucoside, Caprylyl Glucoside, Caprylyl/Capryl Glucoside, Lauryl Glucoside et Myristyl Glucoside sont contenus, Cetearyl Glucoside étant notamment préféré.

**5.** Émulsion selon l'une quelconque des revendications précédentes, **caractérisée en ce que** la fraction massique de l'ensemble des glucosides d'alcools gras dans l'émulsion est de 0,1 à 5,0 % en poids, de préférence de 0,8 à 3,5 % en poids, de préférence de 1,0 à 2,5 % en poids et de manière particulièrement préférée de 1,2 à 2,2 % en poids, chaque fois par rapport au poids total de l'émulsion.

**6.** Émulsion selon l'une quelconque des revendications 3 à 5, **caractérisée en ce que** la teneur en Cetearyl Glucoside est de 0,1 à 5,0 % en poids, de préférence de 0,8 à 3,5 % en poids, de préférence de 1,0 à 2,5 % en poids et de manière particulièrement préférée de 1,2 à 2,2 % en poids, chaque fois par rapport au poids total de l'émulsion.

**7.** Émulsion selon l'une quelconque des revendications précédentes, **caractérisée en ce que** le système émulsifiant comprend du Sorbitan Stearate, la teneur totale en Sorbitan Stearate étant avantageusement de 0,1 à 1,5 % en poids, de préférence de 0,15 à 1,0 % en poids et en particulier de 0,2 à 0,8 % en poids, par rapport au poids total de l'émulsion.

**8.** Émulsion selon l'une quelconque des revendications précédentes, **caractérisée en ce que** le système émulsifiant comprend du Disodium Cetearyl Sulfosuccinate, la teneur totale en Disodium Cetearyl Sulfosuccinate étant avantageusement de 0,01 à 0,5 % en poids, de préférence de 0,02 à 0,2 % en poids et en particulier de 0,04 à 0,10 % en poids, par rapport au poids total de l'émulsion.

**9.** Émulsion selon l'une quelconque des revendications précédentes, **caractérisée en ce que** le système émulsifiant comprend du Glyceryl Stearate, la teneur totale en Glyceryl Stearate étant avantageusement de 0,1 à 1,8 % en poids, de préférence de 0,5 à 1,5 % en poids et en particulier de 0,7 à 1,2 % en poids, par rapport au poids total de l'émulsion.

**10.** Émulsion selon l'une quelconque des revendications précédentes, **caractérisée en ce que** la teneur en alcool myristylique est de 0,2 à 6,5 % en poids, de préférence de 0,5 à 6,0 % en poids, de préférence de 1,5 à 5,0 % en poids, de préférence de 2,5 à 4,5 % en poids et de manière particulièrement préférée de 3,0 à 4,0 % en poids, chaque fois par rapport au poids total de l'émulsion.

**11.** Émulsion selon l'une quelconque des revendications précédentes, **caractérisée en ce que** les structures liquides cristallines dans l'émulsion se présentent sous une structure globulaire.

**12.** Émulsion selon l'une quelconque des revendications précédentes, **caractérisée en ce qu'**en tant que polysaccharides, Zea Mays Starch, Sodium Hydroxypropyl Starch Phosphate, Sodium Carboxymethyl Starch, Hydroxypropyl Starch Phosphate, Tapioca Starch, Potato Starch Modified et/ou Distarch Phosphate sont contenus, Hydroxypropyl Starch Phosphate et Distarch Phosphate étant préférés, Hydroxypropyl Starch Phosphate étant particulièrement préféré.

13. Émulsion selon l'une quelconque des revendications précédentes, **caractérisée en ce que** la teneur en amidons dans l'émulsion est de 0,05 à 4 % en poids, de préférence de 0,1 à 2,5 % en poids, de préférence de 0,15 à 1,5 % en poids et de manière particulièrement préférée de 0,2 à 1,0 % en poids, par rapport au poids total de l'émulsion.

14. Émulsion selon l'une quelconque des l'une quelconque revendications, **caractérisée en ce que** la teneur totale en composants cireux ayant un point de fusion dans la plage de 25 °C à 43 °C est de 0,5 à 5 % en poids, de préférence de 1,5 à 4,5 % en poids et de manière particulièrement préférée de 2,2 à 3,5 % en poids, par rapport au poids total de l'émulsion.

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 10148825 A1
- DE 19938756 A1
- DE 29924371 U1
- DE 2909087 C2
- CN 109528503 A

**In der Beschreibung aufgeführte Nicht-Patentliteratur**

- Pigmenthaltige sonnenschutzemulsionen vom typ O/W mit verbesserten eigenschaften. RESEARCH DISCLOSURE. KENNETH MASON PUBLICA-TIONS, 01 July 2000, vol. 435